# EUROPEAN PATENT APPLICATION

(11) **EP 1 036 574 A1**
(43) Date of publication of application: **20.09.2000**
(21) Application number: 00600001.2
(22) Date of filing: 31.01.2000
(51) Int. Cl.: A61N 2/02

(54) **Method and organisation of external heating of metallic elements and implants with the use of a magnetic field**

(30) Priority: 15.02.1999 GR 99100054
(71) Applicant: Diamantopoulos, Leonidas, N. Iraklio Attikis (GR)
(72) Inventor: Diamantopoulos, Leonidas, N. Iraklio Attikis (GR)
(74) Representative: Petsis, Christos

(57) **Abstract**

The described invention is a system that generates alternating magnetic field of appropriate power and frequency, and then direct it to a human body or animal where a metallic implant (stent) or other metal has been previously implanted. The magnetic field power is continuously controlled by a computer with special software. As the magnetizing force changes periodically, the magnetic flux inside the metal lags behind due to its magnetic retentivity. Therefore, to overcome this resistance, energy is spent which appears on the metal as heat. This heat production regards only metals and leave intermediate tissues and non-metallic materials unaffected. In other words, we manage to heat non-invasively implanted metals up to the desired temperature. Heating of such implanted metals, like stents, is possible to reverse restenosis process in stented vessels. In some metals, heating can change their geometry, property which can be useful in Medicine. Finally, the application of the non-invasive heating could prove to be a new method for treating other modalities, like cancer, etc.

## Description

### Current technology.

Up to now, the temperature at the surface of intravascular intraluminal metallic implants is the same as the surrounding medium, ie. blood, at approximately 37 degrees. There is no current method to increase the local temperature at the site of the metallic implants. Experimental work has shown that increase of temperature of the atheromatous plaque in the vascular tree induces apoptosis, thereby reducing the speed and importance of atherosclerotic development and thus possibly reducing the whole process of atheromatosis. The technology that we are introducing is precisely filling the gap to induce such focal rise in temperature. Based on the properties of metals when they are inside an alternating magnetic field, this method succeeds in heating the metallic implants (for example stents) from a distance of several centimeters without affecting the intermediate tissues. The application of the invention in animals as well as in humans takes place externally without any kind of danger to the subject or without introducing any foreign body into the subject.

### Description:

As shown in plan 1a, when a coil is being ran from a continuous current, it forms around it a magnetic field, the magnetic lines of which enter from the coil edge (A), cross the central axis of the coil, where the maximal flux density exists, and exits from edge (B), circle externally, and reenter from edge (A). When an external metallic cylinder is placed in the course of magnetic flux, the density of the fields' magnetic lines, is altered. The degree of alteration of the magnetic lines, depends, amongst others, on the magnetic permeability of the metal inside it. Metal with high magnetic permeability increase a lot the local density of the magnetic lines. However, one must ask oneself what would happen if alternating electrical current of specific frequency was applied. The result is the formation of an alternating magnetic field. The frequency of the field equals the frequency of the current. If we put again the metallic cylinder into the field, then on it appears a current of energy loss which is called hysteresis, and is due to the magnetic "memory" of the metallic cylinder. In plan 1b, one can appreciate 2 graphical plots; the left one shows the density of the magnetic lines inside the metal, that can be increased up to a certain point, after which a plateau forms, which is the point when the metal is saturated. The right plot shows the changes of the magnetic flux density in relation to the changes of the magnetic force: starting from point A (zero magnetic flux) the flux reaches the maximal value at point B, which corresponds to the maximal magnetic force. As the magnetic force is reduced, the same happens to the flux, but it does not reach zero simultaneously with the magnetic force at point D. As the field is reversed, the magnetic force continues to the opposite direction, and brings the flux at point C. As the force reaches zero, the flux once more lags behind. As a result, an opposite magnetic force is necessary to overcome the residual magnetism of the metallic cylinder, which is called coercive force. This leads to energy loss from the magnetic field on the magnetic cylinder, which appears as heat, increasing the cylinders' temperature . Since this energy appears as heat in each period of the field, the temperature of the cylinder increases rapidly.

Further in this text, the coil will be called "thermal induction coil".

In plan 4, the basic plan of the invention is shown. The thermal induction coil L is connected in parallel with a vacuum capacitor C to form a Thomson circuit (LC). In this way, we create an alternating current of high -but controlled- frequency, which is essential for the operation of the thermal induction coil. The synchronized delivery of electrical energy to the Thomson circuit is achieved by using a power triode electronic tube type 3CX5,000H3 (Eimac, USA).

The voltage +Vcc is 8 KV and is filtered from RF return by the capacitors C3 and C4. Each of them consists of 4 ceramic capacitors 4,7 nF/3KV. Next the voltage passes through the RFC choke, which consists of 70 turns of enamel wires 1 cm in diameter, and is applied to the LC circuit , and then to the anode of the triode tube. The grid of the tube is connected through a feedback capacitor 680 pF/ 10 KV of porcelain type to the active part of the LC, and on the other hand it has a stable polarization with ground through a resistor 47 KOhm/50W and accepts the negative voltage -Vneg. The tubes' filaments are supplied with alternating voltages 7.5V/80Amp from the transformer TR3, and since they are playing the role of a cathode, they are filtered by capacitors C1,C2=47nF/50V. With this apparatus the triode tube operates as a high power self-oscillator with adjustable frequency. The prototype of the invention drained 25KW of electrical power and produced electrical oscillation of 20 MHz and usable power 18KW, which is converted to a magnetic field at the thermal induction coil L. The prototype of the invention was constructed in an air-shielded, closed aluminum chassis for the creation of high air pressure at the tube compartment that is air-cooled. The air compression is achieved with 2 powerful air compressors while a third one is creating negative pressure at the thermal induction coil compartment so that the air flow at the coil is directed from the outside to the inside zeroing the heat emission due to hot air.

Plan 2 shows the electronic circuit for the power supply of the invention. The 220 V net voltage is being raised from the transformer TR1 which as it is shown on the plan, has a secondary with multiple points 1500/2500/3500/5500 Volts, with a maximum current of 3 Amp. The transfer switch SW1 selects the active secondary circuit and the alternating high voltage is forwarded to an AC to DC converting apparatus. At the primary of TR1 there is a special set-up: in order to confront the peak current when the transformer is switched on, due to the self-induction of the primary circuit, there is a time-delay circuit which puts resistances R1 and R2 in series to the primary for 3 seconds and then bypasses them with a high power relay. The AC to DC conversion is achieved by the diode system 1N5408. The DC is driven to capacitors C1-C2 for smoothening, then through the fuse and the Amp meter it is directed to the rest of the invention as the anode voltage +Vcc. In plan 3, the transformer TR2 converts the 220 net voltage to 350 V/ 2 Amp, which is converted to DC by the diode bridge D21-D24. It is smoothened by the electrolytic capacitors C3-C4 and one choke 10mH. This way, a negative voltage of 490 V is created and is forwarded to the wire potentiometer Rt=25KOhm/50W. This way we create an adjustable negative voltage -Vreg=0-490V.
The prototype was constructed as two connected devices: the power supply and the magnetic generator where the tube, the coil and the corresponding circuits are included ( plans 5 and 6). The connection between the two devices happens with special cables that are inside a PVC tube for security reasons. The power supply is fully controlled by a computer through an interface that is an analog-to-digital and vice-versa converter. In this way, the magnetic field strength and heating time and therefore the temperature of the implant are precisely controlled while the correct operation of the system is monitored.

The computer software was constructed in programming language C++. It provides continuous information on the magnetic field strength, the temperatures of the vital parts of the invention, the operation of air compressors and the estimated temperature of the implant. Also, there is the capability of connecting an infrared camera for the objective evaluation of the implants' temperature. The programming from the invention operator allows full control of the device concerning the time and strength of the magnetic field. In the prototype we tested several thermal induction coils with different turns and diameters. Some of them are shown in plans 5 to 7. Regarding the position of the coil relative to the metallic implant we can describe 2 basic variations of the invention. This patent application covers both variations but also any other variation that regards external heating of metallic implants using magnetic field energy.
A)The coil has a great diameter (>20cm) , resulting the patient's entering the device as in a life tire. In this case (plan 6, plan 7c), the metallic implant (stent) is at the centre of the coil and therefore the heating phenomenon is much more intense because the implant is crossed by many dense field magnetic lines.
B) The coil has a smaller diameter (<20cm) and it is placed in such a way that the stent is along the axial line of the coil and at a distance 1-10 cm from the first turn (plan 5, plans 7a,b).

In both cases the coil is heated during its operation because it is crossed by high frequency current. It is very important to keep the coil temperature within acceptable limits. For this purpose, the invention has a special cooling system of the thermal induction coil which enables continuous flow of cold air which keeps the coil and its surroundings within reasonable temperatures (<60 degrees Celsius). The temperature of the coil and of the tube is continuously monitored by an automated circuit which informs of correct operation and automatically switches of in case of emergency.

However, it is obvious that the correct placement of the coil in regard to the implanted metallic device is of great importance. For this purpose the invention is equipped with a low-power laser beam which always points to the right area of maximal heating.

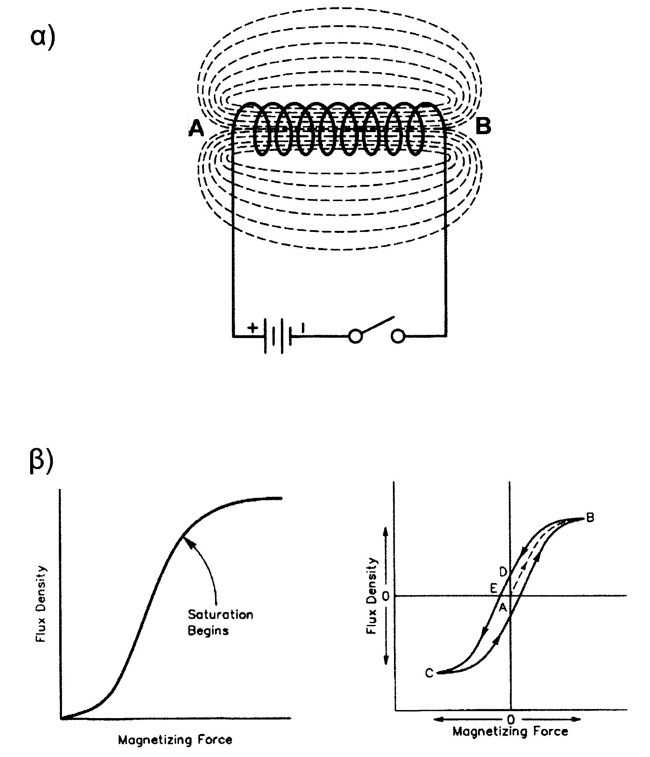

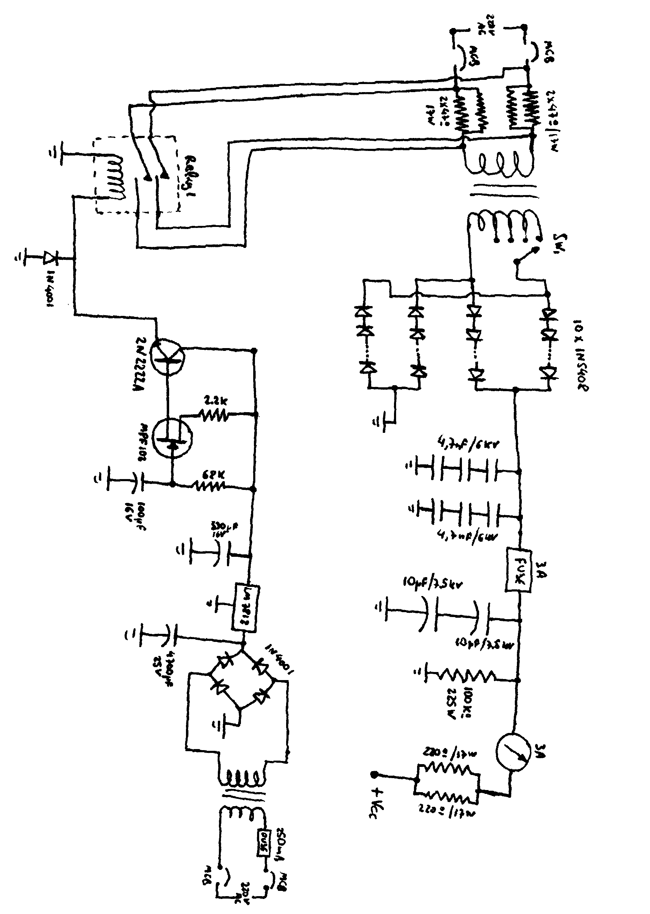

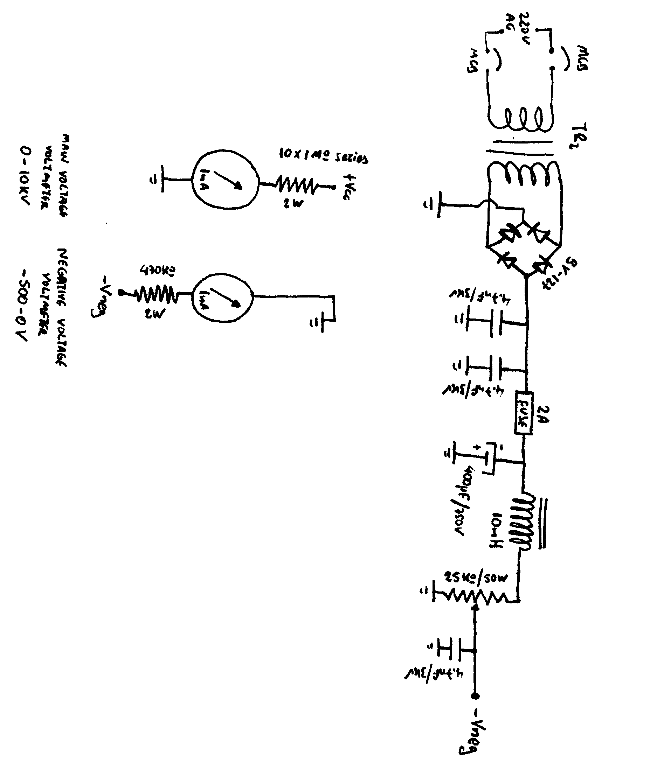

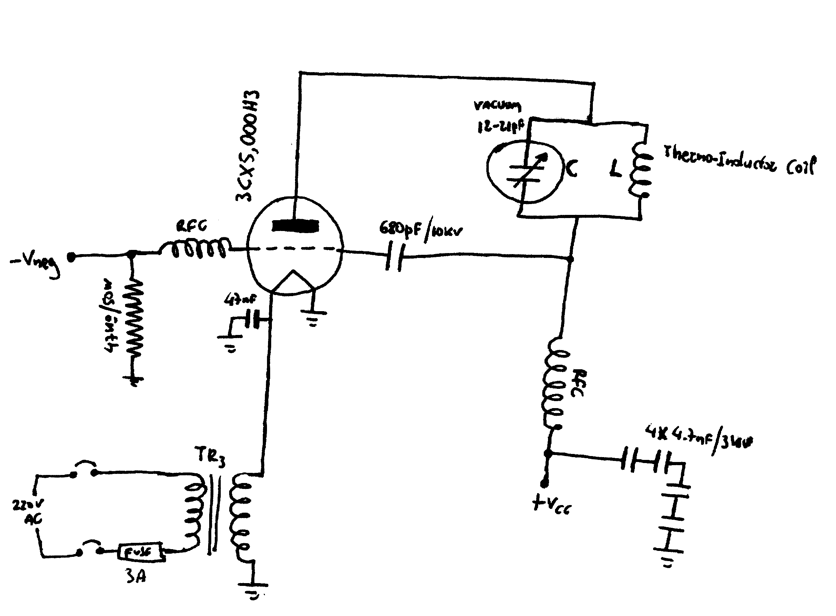

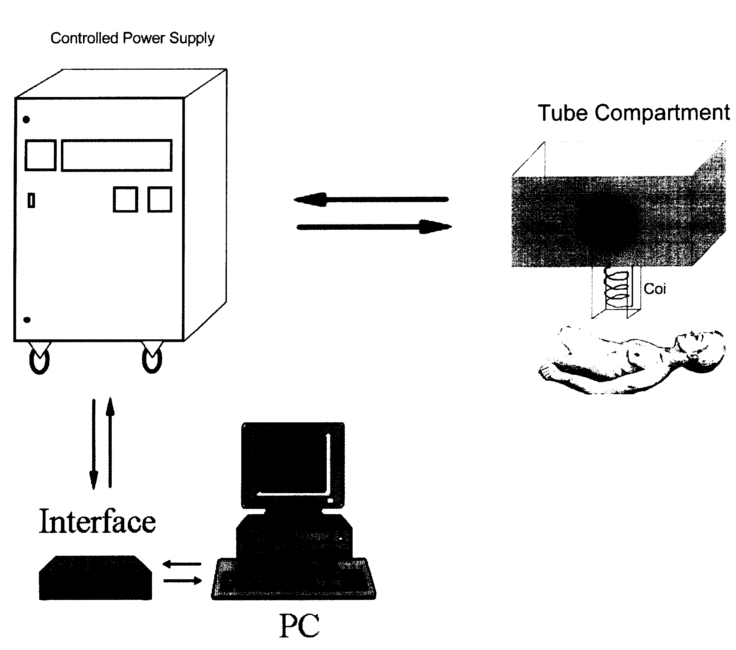

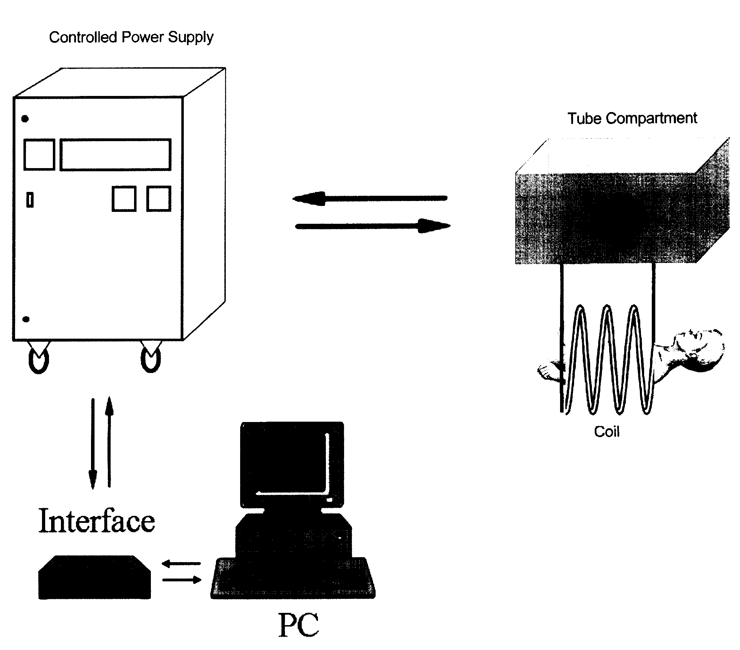

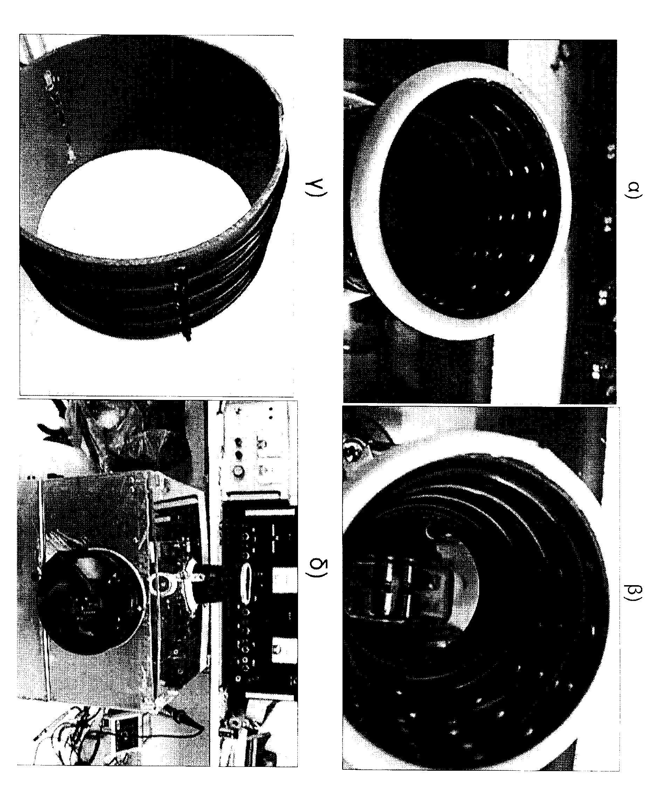

In plans 7 a-d we can see photographs of the tube compartment and the thermal induction coil. Photos a and b show the coil and the PVC cylinder in case we select heating form a distance with a coil <20cm in diameter, while photo 7c shows the coil for heating by placing the patient at the centre of it (coil >20cm). In photo 7d, we can see the air compressor which generates negative pressure inside the PVC cylinder, thus zeroing the hot air emission which potentially would hurt the skin since the coil is hot.

For better understanding of how the device works we will mention an example of applying it on a patient. Supposing our patient has coronary artery disease, and had a stenosis of 90 % at his right coronary artery, for which he underwent angioplasty with stent placement. After the stent implantation, we programmed the patient for example for 10 heating sessions, every seven days. The patient is placed at the thermal induction coil so that his left thorax is at the line of the coil. The invention is switched on, and the stent is heated to 45 degrees Celsius. The same procedure can be repeated quickly and safely as many times as needed.

The previous description is one way of heating metallic implants externally using alternating magnetic fields. The current patent application covers any other way of heating a metallic implant inside the human body or an animal by using magnetic, electromagnetic or other type of radiation.

## Claims

1. System of non-invasive heating of metallic implants consisted of a high-power supply and high-frequency oscillator which leaves its signal to an appropriate coil with the am to produce alternating magnetic field. When the metallic implants are exposed in the field, they are heated, depending on the power and frequency of the field, while the intermediate tissues do not get affected.

2. System of non-invasive heating of metallic implants (stents) as well as expansion of special thermally expanded stents consisting of alternating magnetic field generator with appropriate power and frequency corresponding to claim 1. The heating is due to the magnetic hysteresis phenomenon which generates heat in metals when they happen to be inside the magnetic lines of a rapidly alternating magnetic field.

3. Method of non-invasive external heating of the atheromatic plaque which is based in stent placement and use of alternating magnetic field. The skin and the rest of the intermediate tissues remain unaffected.

4. Method of non-invasive heating of metallic implants according to the claims 1,2,3 which can help in confronting atheromatic disease, neoplasia, or other diseases and help reducing the risk for restenosis after stent placement.
